# EUROPÄISCHE PATENTSCHRIFT

(11) **EP 3 115 008 B1**
(45) Veröffentlichungstag und Bekanntmachung des Hinweises auf die Patenterteilung: **19.06.2019**
(21) Anmeldenummer: 15176156.6
(22) Anmeldetag: 09.07.2015
(51) Int. Cl.: A61B 17/86, A61B 17/70, A61F 2/30

(54) **KNOCHENVERANKERUNGSELEMENT**
BONE ANCHORING ELEMENT
ÉLÉMENT D'ANCRAGE OSSEUX

(43) Veröffentlichungstag der Anmeldung: 11.01.2017
(73) Patentinhaber: Silony Medical International AG, 8500 Frauenfeld (CH)
(72) Erfinder: Heuer, Frank, 70794 Filderstadt (DE); With, Enrico, 78166 Donaueschingen (DE)
(74) Vertreter: Stolmár & Partner Patentanwälte PartG mbB

(56) Entgegenhaltungen:
- EP-A1- 2 476 386
- DE-A1-102004 027 881
- US-A1- 2008 015 596
- US-A1- 2011 172 718
- US-A1- 2012 215 263

## Beschreibung

### Gebiet der Erfindung

Die Erfindung betrifft ein Knochenverankerungselement und ein Verfahren zum Herstellen des Knochenverankerungselements.

### Stand der Technik

Knochenverankerungselemente werden beispielsweise verwendet, um Wirbelfehlstellungen in der Wirbelsäule zu korrigieren. Üblicherweise bestehen diese aus einem Aufnahmeelement, in das eine Knochenschraube eingesetzt wird, die dann in die Wirbelsäule eingeschraubt wird. Mit Hilfe eines zweiten Knochenverankerungselements und eines Querstabs, der die beiden Knochenverankerungselemente verbindet, kann dann eine bestimmte Position der zwei miteinander über die Knochenverankerungselemente verbundenen Wirbel ermöglicht werden.

Bei den üblichen Knochenverankerungselementen weist der Schraubenschaft einen geringeren Durchmesser auf, als der Schraubenkopf, da der Schraubenkopf im Aufnahmeelement gelagert wird und die Schraube so nicht durch das Aufnahmeelement hindurchfallen kann. Ein Schraubenschaft, der einen größeren Durchmesser als der Schraubenkopf aufweist, würde auch nicht durch die Bohrung im Aufnahmeelement passen, da er dann auch zu groß für die Bohrung im Aufnahmeelement wäre. Wenn man also eine Schraube mit einem größeren Knochengewinde verwenden will, wäre die naheliegende Lösung, den Schraubenkopf und das Aufnahmeelement einfach entsprechend zu vergrößern. Das Problem hierbei ist, dass solch große Aufnahmeelemente für den Patienten im Alltag unangenehmer und beschwerlicher sind. Es sollte daher vermieden werden, die Größe des Aufnahmeelements und die Größe des Schraubenkopfs zu vergrößern. Die gängige Ausgestaltung für Pedikelschrauben ist daher nicht verwendbar, wenn eine Knochenverankerungsschraube mit einem Gewinde verwendet werden soll, das einen größeren Außendurchmesser aufweist als der Schraubenkopf bzw. der Bohrungsdurchmesser des Aufnahmeelements.

Diese Problematik wird im Stand der Technik bisher nicht angesprochen, wobei es aber schon Knochenverankerungselemente gibt, bei denen

Die EP 2 462 889 A1 und auch die US 8,377 101 B2 zeigen Ausführungsformen, bei denen ein Hilfselement über den Schraubenkopf gespannt wird und dieses dann in das Aufnahmeelement eingesetzt wird. Bei einer solchen Lösung könnte die Bohrung im Aufnahmeelement groß genug ausgeführt werden, so dass ein größeres Gewinde hindurchgelangen kann oder sogar nicht durch die Bohrung im Aufnahmeelement geschoben werden muss. Diese Lösung ist jedoch insbesondere deshalb nachteilig, weil das Montieren des Knochenelements aufwändiger ist. Außerdem wirken beim Festziehen der Schraube und beim Fixieren des Knochenverankerungselements Kräfte auf das Aufnahmeelement, die dieses auseinander drücken. Daher kann ein mehrteiliges Aufnahmeelement auch eine Schwächung der Struktur bedeuten, was wiederum ein Risiko für die Stabilität des Knochenverankerungselements und möglicherweise nachteilig für den Patienten ist.

Ebenfalls ist es eine Möglichkeit, die Schraube zweiteilig auszuführen. Solche Ausgestaltungen sind beispielsweise in den Druckschriften EP 1 443 866 B1 oder der US 6,187,005 B1 gezeigt, ohne jedoch auf die Problematik des größeren Schraubendurchmessers einzugehen. In solchen Knochenverankerungselementen ist beispielsweise im Schraubenschaft ein Innengewinde in einer Bohrung und am Schraubenkopf ein Vorsprung mit einem Außengewinde vorgesehen (oder umgekehrt), so dass der Schraubenkopf von oben in das Aufnahmeelement eingesetzt und der Schraubenschaft auf den Schraubenkopf aufgeschraubt werden kann. Bei diesen Ausführungsformen wird zuerst der Schraubenschaft eingeschraubt und erst danach das Aufnahmeelement und der Schraubenkopf befestigt.

Das Problem bei den beiden oben genannten Ausführungsformen ist jedoch, dass diese Knochenverankerungselemente mehrteilig ausgebildet sind und erst am Anwendungsort montiert werden (was einen Mehraufwand für den Anwender bedeutet), jedenfalls aber demontiert werden können. Um sicherzustellen, dass nur die Einzelteile eines bestimmten Knochenverankerungselements verwendet werden, müssen alle Einzelteile vom Hersteller gekennzeichnet werden. Eine solche Kennzeichnung der Einzelteile ist bei der Herstellung ebenfalls recht aufwändig.

Die US 2011/172718 A1 offenbart eine Knochenschraube, die einen länglichen Schaft aufweist, der sich in Längsrichtung zwischen einem proximalen Ende und einem gegenüberliegenden distalen Ende erstreckt. Die Welle begrenzt einen ersten Durchgang, der sich zumindest teilweise zwischen dem proximalen Ende und dem distalen Ende erstreckt. Der Schaft besteht aus einem strahlendurchlässigen Material. Ein Kern ist innerhalb des ersten Durchgangs der Welle angeordnet. Der Kern kann aus einem strahlendurchlässigen oder strahlenundurchlässigen Material bestehen. Ein Kopf ist entweder integral mit dem proximalen Ende des Schafts oder dem proximalen Ende des Kerns ausgebildet oder befestigt. Der Kopf kann auch einen zweiten Durchgang begrenzen, der sich durch den Kopf erstreckt und mit dem ersten Durchgang ausgerichtet ist.

Die US 2008/015596 A1 offenbart eine Knochenankeranordnung mit einem großen Durchmesser zum Verbinden eines Wirbelsäulenverbindungselements mit dem Knochen. Die Anordnung umfasst ein Aufnahmeelement zum Aufnehmen des Wirbelsäulenverbindungselements, ein Knocheneingriffselement und eine Kernwelle zum Verbinden des Aufnahmeelements mit der Knocheneingriffshülse.

Die US 2012/215263 A1 offenbart eine verlängerbare Pedikelschraubenkupplungsvorrichtung. Die Einstellung der Länge der Pedikelschraube wird durch den Eingriff voneinem Gewinde durchgeführt, indem der Schraubenkopf in den Schraubenschaft geschraubt wird und dann beim Verankern mit Knochenzement fixiert wird.

Die EP 2 476 386 A1 offenbart dynamische Knochenfixationselemente und ein chirurgisches Verfahren zur Stabilisierung von Knochen- oder Knochenfragmenten. Die dynamischen Knochenfixierungselemente umfassen eine Knocheneingriffskomponente und eine Lastträger-Eingriffskomponente. Die Knocheneingriffskomponente umfasst vorzugsweise eine Vielzahl von Fäden zum Eingreifen in den Knochen eines Patienten und ein Lumen. Die Lastträger-Eingriffskomponente umfasst vorzugsweise einen Kopfabschnitt zum Eingriff mit einem Lastträger (z.B. Knochenplatte) und einem Wellenabschnitt. Der Schaftabschnitt erstreckt sich vorzugsweise zumindest teilweise in das Lumen. Zumindest ein Teil einer Außenfläche des Schaftabschnitts ist von mindestens einem Teil einer Innenfläche des Lumens über einen Spalt beabstandet, so dass sich der Kopfabschnitt in Bezug auf die Knocheneingriffskomponente bewegen kann. Das distale Ende des Schaftabschnitts ist vorzugsweise mit dem Lumen gekoppelt.

### Darstellung der Erfindung

Aufgabe der vorliegenden Erfindung ist daher, ein Knochenverankerungselement bereitzustellen, das es zum Einen ermöglicht eine Schraube mit einem Gewinde zu verwenden, das breiter ist als der Schraubenkopf (und damit breiter als die Durchgangsbohrung im Aufnahmeelement) und dennoch die oben genannten Probleme vermeidet.

Ein erfindungsgemäßes Knochenverankerungselement umfasst ein Aufnahmeelement zum Aufnehmen einer Knochenschraube, wobei das Aufnahmeelement eine Durchgangsbohrung, einen Sitz zum Lagern eines Schraubenkopfes und zwei nach oben gerichtet Schenkel aufweist, eine Knochenschraube, die einen Schraubenkopf und einen Schraubenschaft aufweist, wobei der Schraubenkopf einen Vorsprung und der Schraubenschaft eine Aufnahme für den Vorsprung umfasst, und wobei der Schraubenkopf und der Schraubenschaft an der Aufnahme und dem Vorsprung miteinander gefügt sind, wobei an der Aufnahme (38) und/oder dem Vorsprung (32) Kanäle (29) vorgesehen sind, die zur Schweißgasabfuhr vorgesehen sind. Mit Fügen sind Verbindungsverfahren gemeint, die aus zwei einzelnen Elementen ein einzelnes, nicht mehr zu teilendes Element machen, wie beispielsweise Verpressen (Krimpen), Nieten oder insbesondere Kleben und Schweißen (Laserschweißen, Reibschweißen). Überraschenderweise sind die Fügeverfahren und insbesondere das Schweißen ausreichend stabil, um den Einsatz der Schraube in der Medizin zu gewährleisten. Obwohl im Stand der Technik bisher davon ausgegangen wurde, dass das Fügen der Knochenschraube zu einer für den Einsatz zu unzureichender Qualität führt hat sich jedoch herausgestellt, dass eine gefügte Schraube den hohen Qualitätsansprüchen für den operativen Einsatz genügt. Daher kann eine Knochenschraube verwendet werden, die zum Einen ein größeres Gewinde aufweist als die Durchgangsbohrung im Aufnahmeelement besitzt und zum Anderen durch den Anwender nicht mehr geöffnet werden kann.

An der Oberfläche der Schraube konzentrieren sich bei Schraubenbelastung die Zugspannungen. Um eine höhere Sicherheit der Verbindung zwischen Schraubenkopf und Schraubenschaft zu gewährleisten ist es daher vorteilhaft, die Verbindung möglichst nah an die Schraubenachse zu bringen, weswegen insbesondere Laserschweißen und Reibschweißen bevorzugte Fügeverfahren sind.

An der Aufnahme und/oder dem Vorsprung sind Kanäle vorgesehen, die für den Schweißgasabfluss verwendet werden, wenn als Fügeverfahren Schweißen und insbesondere Laserschweißen verwendet wird. Die Kanäle sind vorzugsweise als Durchgangsbohrungen im Schraubenschaft und/oder als Nuten an der Oberfläche am Schraubenschaft und/oder dem Vorsprung ausgebildet. Insbesondere die Qualität beim Schweißen von Schraubenkopf und Schraubenschaft wird dadurch erhöht. Insbesondere Kanäle an der Oberfläche des Schraubenschafts bzw. des Vorsprungs sind vorzuziehen, da diese eine bessere Abfuhr des Schweißgases ermöglichen. Insbesondere sind diese Kanäle an der Oberfläche entlang einer Längsachse der Knochenschraube ausgebildet.

Die Aufnahme im Schraubenschaft und der Vorsprung sind zumindest abschnittsweise konisch ausgebildet. Diese Ausbildungsart begünstigt das Fügen von Schraubenkopf und Schraubenschaft.

Ferner ist es bevorzugt, dass im oberen Bereich an der Aufnahme und/oder dem Vorsprung eine in Umfangsrichtung umlaufende Nut vorgesehen ist, die vorzugsweise mit den Schweißgasabflußkanälen verbunden ist. Diese umlaufende Nut ist vorzugsweise komplett umlaufend, kann aber auch nur abschnittsweise umlaufend ausgebildet sein. In einer solchen Nut kann beim Verschweißen von Schraubenkopf und Schraubenschaft eine Schweißraupe aufgenommen werden, die beim Schweißen von außen nach innen gebildet wird, und verhindert so eine mögliche innere Kerbentstehung. Dies steigert die Festigkeit der Schraube und die Prozesssicherheit bei der Montage.

Der Schraubenkopf umfasst vorzugsweise einen Hals, auf dem der Vorsprung ausgebildet ist. Dadurch wird es prozesstechnisch einfacher den Schraubenkopf mit dem Schraubenschaft zu fügen, da die Fügestelle einfacher zu erreichen ist. Vorzugsweise ist die Länge des Halses in Axialrichtung mindestens 0,5mm, insbesondere 1mm und weiter bevorzugt 1,5 mm. Der Hals sollte höchstens 2 bis 10mm lang sein.

Ferner kann zur Erhöhung der Festigkeit der Schraube die Fügestelle gestrahlt werden. Dafür wird vorzugsweise ein Kugelstrahlverfahren insbesondere mit Keramikkugeln verwendet. Dadurch wird die Dauerfestigkeit an der Fügestelle erhöht und die durch das Schweißen entstandenen Zugeigenspannungen verringert mit dem Ziel sie in Druckeigenspannungen zu überführen.

Ferner umfasst die Erfindung ein Verfahren zum Herstellen eines Knochenverankerungselements umfassend die Schritte Bereitstellen eines Aufnahmeelements, Einsetzen des Schraubenkopfes in das Aufnahmeelement und Aufsetzen des Schraubenschaftes auf den in das Aufnahmeelement eingesetzten Schraubenkopf und Fügen des Schraubenschaftes mit dem Schraubenkopf.

Insbesondere werden Schraubenschaft und Schraubenkopf mittels Reibschweißen oder Laserschweißen gefügt.

Vorzugsweise wird der gefügte Bereich mit Hilfe eines Strahlmittels, beispielsweise Keramikkugeln oder Stahlkugeln, gestrahlt. Dadurch wird wie schon erwähnt die Dauerfestigkeit der Schraube erhöht, indem die Druckeigenspannungen verbessert und die Zugeigenspannungen durch das Schweißen verringert werden.

### Kurze Beschreibung der Figuren

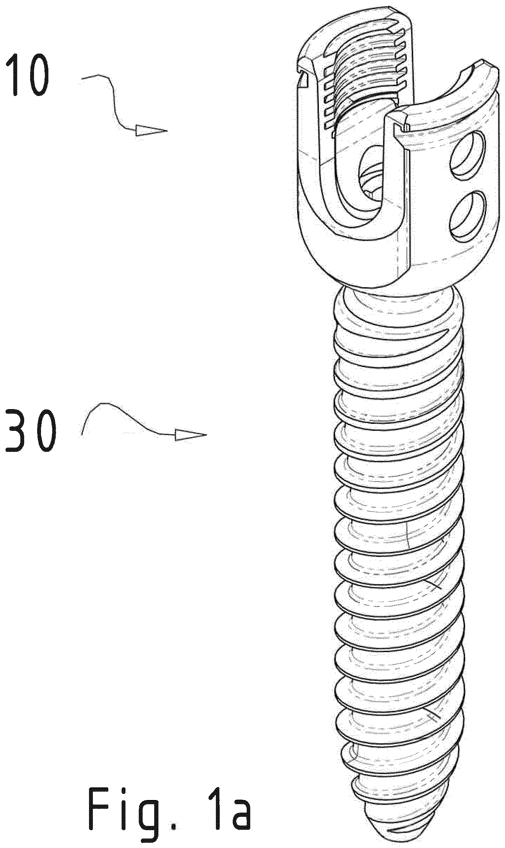

- Figur 1a: zeigt eine isometrische Ansicht eines erfindungsgemäßen Knochenverankerungselements;

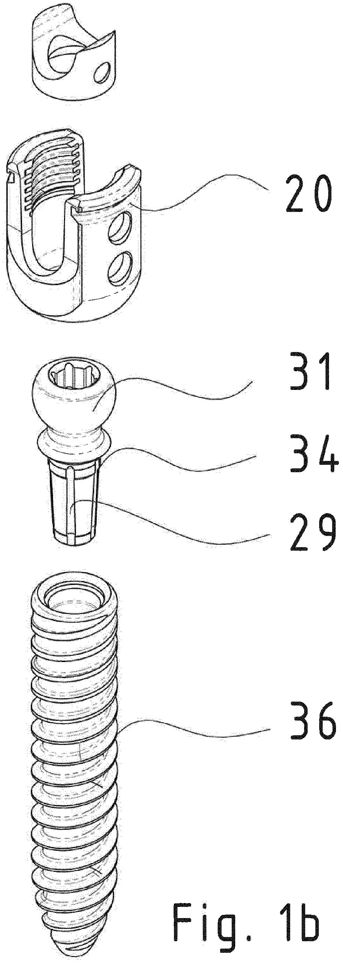

- Figur 1b: zeigt Explosionsansicht des Knochenverankerungselements auf Figur 1a;
- Figur 2a: zeigt eine Schnittzeichnung eines erfindungsgemäßen Knochenverankerungselements mit einer Vergrößerung an der Fügestelle; und
- Figur 2b: zeigt eine geschnittene Explosionsansicht des Knochenverankerungselements auf Figur 2a.

### Bevorzugte Ausführungsform der Erfindung

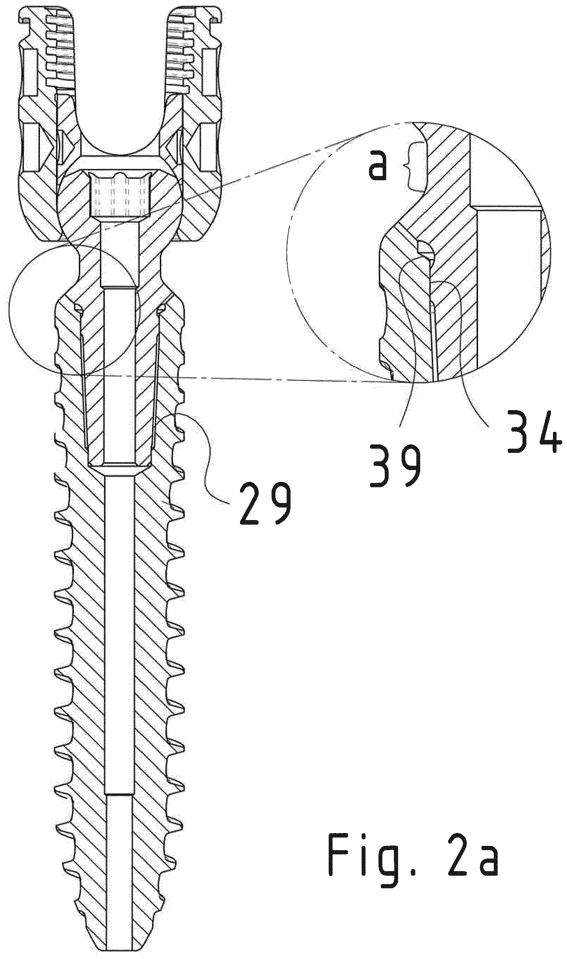

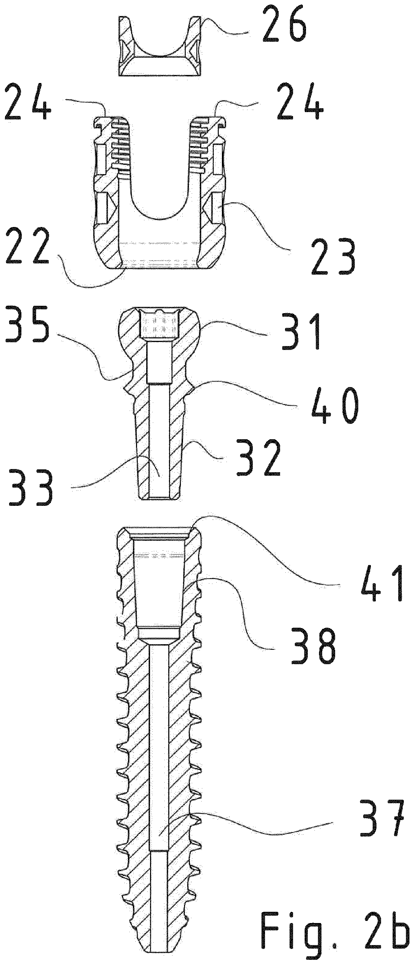

Wenn im Folgenden von "oben" und "unten" die Rede ist, wird dabei in Bezug auf Figur 2a bzw. 2b genommen. Ferner beschreibt Axialrichtung eine Richtung entlang der Achse der Knochenschraube (in Figur 2a also von unten nach oben oder umgekehrt), eine Radialrichtung (eine Richtung senkrecht zur Axialrichtung) und Umfangsrichtung (eine Richtung um die Längsachse herum).

Ein erfindungsgemäßes Knochenverankerungselement 10 umfasst im Wesentlichen ein Aufnahmeelement 20 und eine Knochenschraube 30. Das Aufnahmeelement 20 weist eine Durchgangsbohrung auf, und ist vorzugsweise im Wesentlichen U-förmig im Querschnitt. Am unteren Ende des Aufnahmeelements 20 ist ein Sitz 22 ausgebildet, in dem der Schraubenkopf 31 im montierten Zustand des Knochenverankerungselements lagert. Ferner weist das Aufnahmeelement zwei Schenkel 24 auf, an dessen oberem Rand ein Gewinde (innen oder außen) vorgesehen ist, mit dem dann ein Querstab (nicht gezeigt) mittels einer Madenschraube oder einer Mutter auf das Druckstück 26 gedrückt wird und die Schraube letztlich in der Endstellung fixiert. Die seitlichen Bohrungen 23 dienen zum Verpressen des Druckstücks, wenn das Knochenverankerungselement 10 fertig montiert ist, so dass die Schraube 30 nicht mehr aus dem Aufnahmeelement 20 herausgenommen werden kann oder herausfallen kann.

Die Knochenschraube 30 umfasst einen Schraubenkopf 31 und einen Schraubenschaft 36. Der Schraubenschaft ist zumindest abschnittsweise mit einem Knochengewinde versehen, das eingängig oder auch abschnittsweise oder komplett mehrgängig ausgebildet sein kann. Im Schraubenschaft können Bohrungen (nicht gezeigt) vorgesehen sein, die zum Befestigen einer implantierten Schraube mit Knochenzement dienen. Der Schraubenschaft kann eine Durchgangsbohrung 37 aufweisen, die beispielsweise hilfreich zur Positionierung der Schraube mittels eines Führungsdrahts sein kann.

In einem oberen Bereich des Schraubenschafts ist zumindest abschnittsweise eine Aussparung 38 vorgesehen, die insbesondere konisch ausgebildet ist und zur Aufnahme eines Vorsprungs 32 des Schraubenkopfes 31 verwendet wird. Die Aussparung 38 kann Kanäle an der Oberfläche aufweisen, die sich insbesondere in axialer Richtung an der Oberfläche erstrecken (entsprechend den nachfolgend erläuterten Kanälen 29 am Vorsprung 32). Der Schraubenschaft kann aber auch im oberen Bereich Durchgangsbohrungen aufweisen, die ebenso wie die Nuten an der Oberfläche im Aufnahmebereich 38 als Auslass für Schweißgas verwendet werden können. Ebenfalls kann eine oder mehrere in Umfangsrichtung verlaufende Nut 39 vorgesehen sein, die zur Aufnahme einer Schweißraupe dient, wenn Schraubenkopf und Schraubenschaft durch Schweißen gefügt werden.

Der Schraubenkopf kann ebenfalls eine Durchgangsbohrung 33 aufweisen, die mit der Durchgangsbohrung 37 des Schraubenschafts ausgerichtet ist, so dass eine durchgängige Durchgangsbohrung entsteht. An der Oberfläche des Vorsprungs 32 des Schraubenkopfs 31 können ebenfalls Kanäle 29 in Form von Nuten ausgebildet sein. Ebenso können eine oder mehrere in Umfangsrichtung verlaufende Nuten 39 am Vorsprung ausgebildet sein. Vorzugsweise umfasst der Schraubenkopf 31 einen Hals 35, der weiter vorzugsweise einen Kragen 40 umfasst. Dieser Kragen 40 ist insbesondere einen Radialrichtung hervorstehender Anschlag, der mit einem Komplementär oberen Ende 41 des Schraubenschafts 36 in Kontakt steht. Der Hals kann vom Kragen bis zur Unterkante des Schraubenkopfes in Axialrichtung mindestens 0,5mm, insbesondere 1mm und weiter bevorzugt 1,5 mm, weiter vorzugsweise aber höchstens 2 bis 10mm lang sein. In Figur 2a ist der Hals mit dem Bezugszeichen a markiert.

Ferner kann der Vorsprung einen oder mehrere ringartige Erhöhungen 34 aufweisen, die an der inneren Oberfläche der Aufnahme 38 des Schraubenschafts anliegen. So kann gewährleistet werden, dass beispielsweise beim Reibschweißen vorbestimmt definierte Abschnitte des Vorsprungs mit der Innenfläche der Aufnahme 38 in Kontakt liegen und so die Schweißstellen beim Kaltschweißen klar definiert werden können. Solche ringförmigen Vorsprünge können zusätzlich oder stattdessen an der Innenseite der Aufnahme 38 vorgesehen sein.

Um eine Übertragung des Drehmoments im Einsatz zu erleichtern, können der Vorsprung 32 und die Aufnahme 38 jeweils einen komplementären polygonalen Querschnitt aufweisen oder mit Rippen versehen sein, die sich in entsprechenden Aussparungen am jeweils anderen Element (Schraubenkopfvorsprung oder Schraubenschaftaufnahme) beim Eindrehen abstützen und Kräfte übertragen können. Im Prinzip können der Vorsprung und die Aufnahme auch mit einem Gewinde versehen sein, was aber den Herstellungsaufwand etwas erhöht.

Für die Herstellung des Knochenverankerungselements wird der Schraubenkopf in die Aufnahme des Aufnahmeelements eingesetzt, so dass der Kopf im Sitz 22 des Aufnahmeelements 20 liegt. Dann wird der Schraubenschaft auf den Schraubenkopf gesetzt, so dass der Vorsprung 32 in die Aufnahme 38 eingesetzt wird und Schraubenkopf und Schraubenschaft miteinander gefügt. Dies wird erreicht, indem die beiden mittels eines Spezialklebers, durch Verpressen oder Krimpen oder auch durch Verschweißen des Schraubenschaft und Schraubenkopf miteinander vereint werden. Insbesondere Verkleben und Verschweißen sorgen dafür, dass aus den zwei einzelnen Teilen Schraubenkopf und Schraubenschaft eine einstückige Knochenschraube wird, die aus einem übergangslosen Material besteht. Um die dabei entstehenden Schweiß- oder auch Klebergase abzuführen und eine mögliche Schweißraupe aufzunehmen sind dafür entsprechende Kanäle vorgesehenen (Nuten an der Oberfläche oder Durchgangsbohrungen), so dass an der Schraube keine Kerbwirkung an der Schweißnahtinnenseite entstehen können. Abschließend kann die Fügestelle dann gestrahlt werden, insbesondere Kugelgestrahlt, wobei vorzugsweise Keramik- oder Metallkugeln verwendet werden. Dadurch werden die durch das Fügen entstandenen Spannungen verringert und Druckeigenspannungen aufgebaut, die Festigkeit der Fügestelle merkbar erhöhen und so die Qualität der Schrauben verbessern.

## Patentansprüche

1. Knochenverankerungselement (10) umfassend
ein Aufnahmeelement (20) zum Aufnehmen einer Knochenschraube (30), wobei das Aufnahmeelement (20) eine Durchgangsbohrung, einen Sitz (22) zum Lagern eines Schraubenkopfes (31) und zwei nach oben gerichtete Schenkel aufweist;
eine Knochenschraube (30), die einen Schraubenkopf (31) und einen Schraubenschaft (36) aufweist, wobei der Schraubenkopf (31) einen Vorsprung (32) und der Schraubenschaft (36) eine Aufnahme (38) für den Vorsprung (32) umfasst;
wobei der Schraubenkopf (31) und der Schraubenschaft (36) an der Aufnahme (38) und dem Vorsprung (32) miteinander gefügt sind;
**dadurch gekennzeichnet, dass**
an der Aufnahme (38) und/oder dem Vorsprung (32) Kanäle (29) vorgesehen sind, die zur Schweißgasabfuhr vorgesehen sind.

2. Knochenverankerungselement (10) nach Anspruch 1, bei dem die Kanäle (29) als Durchgangsbohrungen im Schraubenschaft und/oder als Nuten an der Oberfläche ausgebildet sind.

3. Knochenverankerungselement (10) nach Anspruch 1 oder 2, bei dem die Kanäle (29) sich an der Oberfläche entlang einer Längsachse der Knochenschraube (30) erstrecken.

4. Knochenverankerungselement (10) nach einem der vorhergehenden Ansprüche, bei dem die Aufnahme (38) und der Vorsprung (32) zumindest Abschnittsweise konisch ausgebildet sind.

5. Knochenverankerungselement (10) nach einem der vorhergehenden Ansprüche, bei dem im oberen Bereich an der Aufnahme (38) und/oder dem Vorsprung (32) eine in Umfangsrichtung umlaufende Nut vorgesehen ist.

6. Knochenverankerungselement (10) nach einem der vorhergehenden Ansprüche, bei dem der Schraubenkopf (31) einen Hals (35) umfasst, auf dem der Vorsprung (32) ausgebildet ist.

7. Knochenverankerungselement (10) nach einem der vorhergehenden Ansprüche, bei dem die Fügestelle gestrahlt ist.

8. Verfahren zum Herstellen eines Knochenverankerungselements (10) nach einem der vorhergehenden Ansprüche, umfassend die Schritte:
Bereitstellen eines Aufnahmeelements (20), eines Schraubenschaftes (36) und eines Schraubenkopfes (31), wobei der Schraubenkopf (31) einen Vorsprung (32) und der Schraubenschaft (36) eine Aufnahme (38) für den Vorsprung (32) umfasst;
wobei an der Aufnahme (38) und/oder dem Vorsprung (32) Kanäle (29) vorgesehen sind;
Einsetzen des Schraubenkopfes (31) in das Aufnahmeelement (20);
Aufsetzen des Schraubenschaftes (36) auf den in das Aufnahmeelement (20) eingesetzten Schraubenkopf (31); und
Fügen des Schraubenschaftes (38) mit dem Schraubenkopf (31).

9. Verfahren nach Anspruch 8, bei dem das Fügen mittels Reibschweißen oder Laserschweißen durchgeführt wird.

10. Verfahren nach einem der Ansprüche 8 oder 9, bei dem der gefügte Bereich mit Hilfe eines Strahlmittels gestrahlt wird.

## Claims

1. A bone anchoring element (10) comprising
a receiving member (20) for receiving a bone screw (30), the receiving member (20) having a through bore, a seat (22) for supporting a screw head (31), and two upwardly directed branches;
a bone screw (30) having a screw head (31) and a screw shank (36), the screw head (31) comprises a protrusion (32) and the screw shank (36) comprises a recess (38) for the protrusion (32);
**characterized in that**
on the recess (38) and/or the protrusion (32) channels (29) are provided, which are provided for welding gas discharge.

2. Bone anchoring element (10) according to claim 1, wherein the channels (29) are formed as through holes in the screw shaft and/or as grooves on the surface.

3. Bone anchoring element (10) according to claim 1 or 2, wherein the channels (29) extend on the surface along a longitudinal axis of the bone screw (30).

4. Bone anchoring element (10) according to any one of the preceding claims, wherein the recess (38) and the projection (32) are conically formed at least in sections.

5. Bone anchoring element (10) according to any one of the preceding claims, wherein a groove encircling in circumferential direction is provided in the upper region on the recess (38) and/or the projection (32).

6. Bone anchoring element (10) according to any one of the preceding claims, wherein the screw head (31) comprises a neck (35), on which the projection (32) is formed.

7. Bone anchoring element (10) according to any one of the preceding claims, wherein the joint location is blasted.

8. Method of producing a bone anchoring element (10) according to any one of the preceding claims, comprising the steps:
Providing a receiving member (20), a screw shank (36) and a screw head (31), wherein the screw head (31) comprises a projection (32) and the screw shank (36) comprises a recess (38) for the projection (32);
wherein channels (29) are provided on the recess (38) and/or the projection (32);
Inserting the screw head (31) into the receiving element (20);
Placing the screw shank (36) on the screw head (31) inserted in the receiving element (20); and
Joining the screw shank (38) with the screw head (31).

9. Method according to claim 8, wherein joining is carried out by friction welding or laser welding.

10. Method according to any one of claims 8 or 9, wherein the joined area is blasted by means of a blasting medium.

## Revendications

1. Élément d'ancrage osseux (10) comprenant
un élément de réception (20) pour recevoir une vis à os (30), l'élément de réception (20) ayant un trou traversant, un siège (22) pour supporter une tête de vis (31) et deux jambes dirigées vers le haut;
une vis à os (30) ayant une tête de vis (31) et une tige de vis (36), la tête de vis (31) comprenant une saillie (32) et la tige de vis (36) comprenant un receptacle (38) pour la saillie (32);
dans lequel la tête de vis (31) et la tige de vis (36) sont joints au réceptacle (38) et à la saillie (32);
**caractérisé en ce que**
sur le réceptacle (38) et/ou la saillie (32) des canaux (29) destinés à l'évacuation du gaz de soudage sont prévus.

2. Elément d'ancrage osseux (10) selon la revendication 1, dans lequel les canaux (29) sont formés sous la forme de trous traversants dans la tige de vis et/ou sous la forme de rainures sur la surface.

3. Elément d'ancrage osseux (10) selon la revendication 1 ou 2, dans lequel les canaux (29) s'étendent sur la surface le long d'un axe longitudinal de la vis à os (30).

4. Elément d'ancrage osseux (10) selon l'une quelconque des revendications précédentes, dans lequel le réceptacle (38) et la saillie (32) sont au moins en section conique.

5. Elément d'ancrage osseux (10) selon l'une quelconque des revendications précédentes, dans lequel dans la région supérieure du réceptacle (38) et/ou de la saillie (32) une rainure circonférentielle est prévue circonférentiellement.

6. Elément d'ancrage osseux (10) selon l'une quelconque des revendications précédentes, dans lequel la tête de vis (31) comprend un col (35) sur lequel la saillie (32) est formée.

7. Elément d'ancrage osseux (10) selon l'une quelconque des revendications précédentes, dans lequel l'articulation est sablée.

8. Procédé de fabrication d'un élément d'ancrage osseux (10) selon l'une des revendications précédentes, comprenant les étapes consistant à:
fournir un élément de réception (20), une tige de vis (36) et une tête de vis (31), la tête de vis (31) comprenant une saillie (32) et la tige de vis (36) comprenant un réceptacle (38) pour la saillie (32);
dans lequel sur le réceptacle (38) et/ou la saillie (32) des canaux (29) destinés à l'évacuation du gaz de soudage sont prévus;
insérer la tête de vis (31) dans l'élément de réception (20);
placer la tige de vis (36) sur la tête de vis (31) insérée dans l'élément de réception (20); et
relier la tige de la vis (38) à la tête de la vis (31).

9. Procédé selon la revendication 8, dans lequel la jonction est réalisée au moyen d'un soudage par friction ou d'un soudage laser.

10. Procédé selon l'une quelconque des revendications 8 ou 9, dans lequel la partie jointe est sablée au moyen d'un agent de sablage.
